# EUROPÄISCHE PATENTANMELDUNG

(11) **EP 2 671 525 A1**
(43) Veröffentlichungstag der Anmeldung: **11.12.2013**
(21) Anmeldenummer: 12171288.9
(22) Anmeldetag: 08.06.2012
(51) Int. Cl.: A61B 17/34, A61F 9/007

(54) **Inzionsmesser**

(71) Anmelder: Oertli-Instrumente AG, 9442 Berneck (CH)
(72) Erfinder: Nyffenegger, Bruno, 9436 Balgach (CH); Sanseverino, Flavio, 9434 Au (CH)
(74) Vertreter: Frischknecht, Harry Ralph

(57) **Zusammenfassung**

Ein Inzisionsmesser (1) für die Ophthalmologie umfasst einen Schaftabschnitt (2) mit einem im Wesentlichen zylindrischen Querschnitt, welcher sich entlang einer Mittelachse (M) erstreckt; und einen Schneidenabschnitt (3), der sich am distalen Ende (4) des Schaftabschnittes (2) an den Schaftabschnitt (2) anschliesst. Der Schneidenabschnitt (3) umfasst eine obere Oberfläche (5) und eine untere Oberfläche (6), wobei die obere Oberfläche (5) und die untere Oberfläche (6) eine erste Schneidkante (7) und eine zweite Schneidkante (8) in einer ersten Ebene (El) bilden, wobei die erste Schneidkante (6) und die zweite Schneidkante (7) im Bereich des distalen Endes (9) des Schneidenabschnittes (3) aufeinander treffen und eine Spitze (10) bilden. Die Spitze (10) ist mit einer Rundung (11) abgerundet ausgebildet.

## Beschreibung

### TECHNISCHES GEBIET

Die vorliegende Erfindung betrifft ein Inzisionsmesser für den Einsatz in der Ophthalmologie gemäss dem Oberbegriff von Anspruch 1.

### STAND DER TECHNIK

Bei Operationen im hinteren Abschnitt des Auges, z.B. Operationen im Glaskörper und an der Netzhaut, wird ein Zutritt für die Instrumente durch die Zone der pars plana vorgenommen. Im heutigen Stand der Operations-Technik erfolgt diese Inzision über ein Trokar-System bzw. über ein Kanülen-System, gemäss welchem ein Trokar bzw. eine Kanüle bzw. ein Führungsrohr in das Auge eingeführt werden. Dabei wird mit einem schneidenden Instrument, insbesondere einem Inzisionsmesser, ein Schnitt (Inzision) gleichzeitig durch Bindehaut (Konjunktiva) und Lederhaut (Sklera) erzielt. Anschliessend wird in diese Inzision ein Führungsröhrchen bzw. ein Trokar bzw. eine Kanüle, das mit oder ohne Dichtung ausgeführt sein kann, gesteckt, um einen geführten Zugang der verschiedenen zur Operationen benötigten Instrumente zu ermöglichen und dabei die Inzision zu schützen.

Dieser Prozess kann in zwei Schritten oder in einem Schritt erfolgen. Im ersten Fall ist das Messer ein getrenntes Teil und das Führungsrohr ist zur geeigneten Handhabung lose auf einem Dorn platziert, der in die Inzision gestossen wird und beim Hinausziehen das Führungsrohr in der Inzision zurücklässt. Im zweiten Fall ist das Führungsrohr direkt auf dem Schaft des Messers platziert und es bleibt beim Zurückziehen des Messers in der Inzision zurück.

Das Inzisionsmesser soll in beiden Fällen vom Operateur zunächst präzise positionierbar sein, dann leicht in die Lederhaut des Auges eindringen, und anschliessend auf seinem ganzen weiteren Eindringweg gut kontrollierbar fortschreiten.

Ein solches Inzisionsmesser ist beispielsweise aus der WO 2010/047984 bekannt. Dieses Messer hat aber eine Reihe von Nachteilen. Einerseits kann das Inzisionsmesser nur sehr schwierig präzise auf der Bindehaut des Auges positioniert werden. Sobald die gewünschte Inzisionsposition leicht verschoben werden soll und bereits ein Kontakt des Messers mit der Bindehaut stattgefunden hat, kann es zu einem Einhängen der Messerspitze auf oder an der Bindehaut kommen, was zu unerwünschten Verletzungen derselben oder von Blutgefässen führen kann. Weiter hat dieses Inzisionsmesser den Nachteil, dass bei der Handhabung vor und beim Erstellen der Inzision, das Messer im Spitzenbereich leicht verformt oder verbogen werden kann, was dann das Eindringen in die Sklera erschwert oder gar verunmöglicht. Dies kann insbesondere bei der versehentlichen Berührung von Metallteilen am Auge wie dem Instrument zum Verschieben der Bindehaut, der Lidsperre, oder schon gesetzen Führorungsrohren mit der Messerspitze leicht passieren,

### DARSTELLUNG DER ERFINDUNG

Ausgehend von diesem Stand der Technik liegt der Erfindung eine Aufgabe zugrunde, ein Inzisionsmesser anzugeben, welches die Nachteile des Standes der Technik überwindet. Insbesondere soll das Inzisionsmesser einfacher auf der Bindhaut vor dem Erstellen der Inzision positionierbar und robuster gegen Beschädigung seiner Spitze sein.

Diese Aufgabe löst der Gegenstand nach Anspruch 1. Demgemäss umfasst ein Inzisionsmesser für die Ophthalmologie einen Schaftabschnitt mit einem im Wesentlichen zylindrischen Querschnitt, welcher sich entlang einer Mittelachse erstreckt, und einen Schneidenabschnitt, der sich am distalen Ende des Schaftabschnittes an den Schaftabschnitt anschliesst, wobei der Schneidenabschnitt eine obere Oberfläche und eine untere Oberfläche umfasst, wobei die obere Oberfläche und die untere Oberfläche eine erste Schneidkante und eine zweite Schneidkante in einer ersten Ebene bilden. Die erste Schneidkante und die zweite Schneidkante treffen im Bereich des distalen Endes des Schneidenabschnittes aufeinander und bilden eine Spitze. Die Spitze ist mit einer Rundung abgerundet ausgebildet.

Die abgerundete Spitze hat dabei den Vorteil, dass das Inzisionsmesser leicht im Kontakt über die Oberfläche des Auges geführt werden kann, ohne dass die Spitze unbeabsichtigt einhängt. Hierdurch hat der Benutzer grosse Vorteile bei der genauen Positionierung des Messers, ohne dass die Bindehaut unabsichtlich und vor allem unnötig verletzt wird. Weiter ist eine erfindungsgemässe verrundete Spitze mechanisch stabiler als eine Spitze ohne die Verrundung, und verringert daher die Notwendigkeit im Fall von Messerspitzenbeschädigungen zusätzliches Operationsmaterial, insbesondere zusätzliche Inzisionsmesser, einsetzen zu müssen.

Bevorzugterweise erstreckt sich die Rundung um ein Zentrum herum, wobei das Zentrum auf der Mittelachse und in der ersten Ebene liegt.

Die Rundung weist bevorzugt einen konstanten Radius oder aber einen variablen Radius auf. Weiter ist die Rundung vorzugsweise konvex ausgebildet.

Vorzugweise weist die Rundung eine Rundungsfläche auf, die sich im Wesentlichen rechtwinklig zur ersten Ebene erstreckt. Die Rundungsfläche begrenzt dabei die Rundung und kann auch als Rundungswand bezeichnet werden. Die Rundungswand bzw. die Rundungsfläche verlaufen dabei senkrecht zur ersten Ebene und erstreckt sich beidseitig zur ersten Ebene. Mit anderen Worten kann auch gesagt werden, dass der Winkel zwischen Rundungsfläche und der ersten Ebene ein rechter Winkel ist.

Bezüglich der geometrischen Ausbildung der Rundungsfläche kann gesagt werden, dass die Rundungsfläche im Bereich der Spitze durch einen virtuellen Zylinder mit kreisrundem oder elliptischem Querschnitt begrenzt wird, wobei die Mittelachse des Zylinders rechtwinklig zur ersten Ebene und zur Mittelachse des Schaftabschnittes steht.

Vorzugsweise erstreckt sich die Rundungsfläche um eine durch besagtes Zentrum hindurchlaufende und zur ersten Ebene rechtwinklig stehende Zentrumsachse. Das Zentrum wäre dann Teil der Mittelachse des besagten virtuellen Zylinders.

Vorzugsweise laufen die obere Oberfläche und die untere Oberfläche bezüglich der ersten Ebene zur Spitze aufeinander zu, wobei sich die Rundung im Wesentlichen senkrecht zur ersten Ebene erstreckt.

Alternativ erstreckt sich die Rundung von besagtem Zentrum kugelförmig oder ellipsoid mit unterschiedlichen Radien, so dass die beiden Schneidkanten und teilweise die beiden Oberfläche durch die Rundung abgerundet sind. Hierbei weist die Rundungsfläche eine Oberfläche auf, die teilweise kugelförmig oder ellipsenförmig ist.

Der Radius der Rundung liegt vorzugsweise im Bereich von 0.01 bis 0.10 mm, besonders bevorzugt im Bereich von 0.01 bis 0.04 mm, und der Durchmesser des Schaftabschnittes liegt vorzugsweise im Bereich von 0.35 bis 0.7 mm.

Vorzugsweise schliessen die Scheitel der oberen Oberfläche und der unteren Oberfläche auf die zweite Ebene, die senkrecht zur ersten Ebene steht, gesehen, einen Winkel ein und verlaufen geneigt zueinander sowie geneigt zur Mittelachse, wobei der Winkel im Bereich von 1° ° bis 20°, vorzugsweise im Bereich von 8° bis 14° verläuft und wobei der Teilwinkel der jeweiligen Oberflächen zur Mittelachse vorzugsweise gleich gross ist.

Vorzugsweise sind die obere Oberfläche und die untere Oberfläche im Querschnitt gesehen konvex gerundet ausgebildet, wobei der Radius in einer Schnittebene, die senkrecht zur besagten ersten Ebene und zur Mittelachse des Schaftabschnittes steht, mit zunehmender Entfernung von der Spitze vorzugsweise grösser wird. Die beiden Oberflächen gehen dann in den Schaftabschnitt über.

Vorzugsweise erstrecken sich die Schneidkanten entlang jeweils einer Geraden, wobei die Gerade winklig zur Mittelachse steht, so dass die erste und die zweite Schneidkante in der ersten Ebene mit einem Winkel im Bereich von 10° bis 30°, vorzugsweise im Bereich von 15° bis 25°, besonders bevorzugt im Bereich von 17° bis 23° zueinander verlaufen.

Vorzugsweise schliessen sich die Geraden und somit die Schneidkanten in einer Ansicht rechtwinklig auf die Ebene gesehen tangential an die Rundung bzw. die Rundungsfläche an.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht eines Inzisionsmesser nach einer ersten Ausführungsform;
- Fig. 2: eine Draufsicht des Inzisionsmessers nach Figur 1 mit einer zusätzlichen Schnittdarstellung entlang der Schnittlinie A-;
- Fig. 3: eine Seitenansicht des Inzisionsmessers nach Figur 2;
- Fig. 4: eine Schnittansicht entlang der Schnittlinie IV-IV nach Figur 3; und
- Fig. 5: eine schematische Detailansicht der Spitze des Inzisionsmessers nach Figur 1 in der Draufsicht; und
- Fig. 6: eine schematische Detailansicht der Spitze des Inzisionsmessers nach Figur 1 in der Seitenansicht.

### BESCHREIBUNG BEVORZUGTER AUSFÜHRUNGSFORMEN

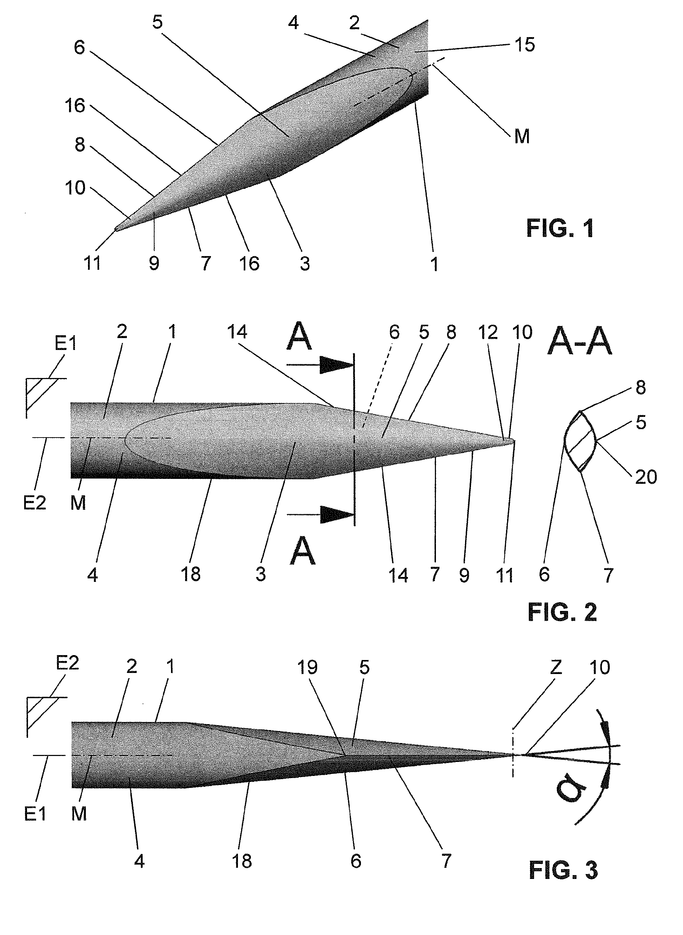

In der Fig. 1 wird eine Ausführungsform eines Inzisionsmessers 1 für die Ophthalmologie gezeigt. Das Inzisionsmesser 1 umfasst hier einen Schaftabschnitt 2 und einen sich dem Schaftabschnitt 2 anschliessenden Schneidenabschnitt 3. Das Inzisionsmesser 1 kann über den Schaftabschnitt 2 mit einem Haltegriff ergriffen werden und über den Schneidenabschnitt 3 kann die Inzision hergestellt werden. Vorzugsweise ist der Haltergriff mit dem Inzisionsmesser verklebt.

Der Schaftabschnitt 2 erstreckt sich im Wesentlichen entlang einer Mittelachse M und weist einen im Wesentlichen zylindrischen Querschnitt auf. Mit anderen Worten kann auch gesagt werden, dass der Schaftabschnitt 2 die Gestalt eines Zylinders mit einer Mantelfläche 15 aufweist. Der Schaftabschnitt 2 umfasst ein distales Ende 4, welchem sich dann der Schneidenabschnitt 3 anschliesst. Gegenüber dem distalen Ende 4 in Richtung der Mittelachse M gesehen, schliesst sich dann ein Handgriff oder andere Elemente, welche von einer Bedienperson ergriffen werden können, an. Die Ausbildung dieser Elemente kann beliebig sein.

Der Schneidenabschnitt 3, der sich am distalen Ende 4 des Schaftabschnittes 2 an den Schaftabschnitt 2 anschliesst umfasst im Wesentlichen eine obere Oberfläche 5 und eine untere Oberfläche 6. Wie die nachfolgende Beschreibung zeigt, verlaufen die beiden Oberflächen 5, 6 winklig oder geneigt zueinander aufeinander zu. In einer ersten Ebene E1 bilden die obere Oberfläche 5 und die untere Oberfläche 6 eine erste Schneidkante 7 und eine zweite Schneidkante 8. Diese beiden Schneidkanten 7, 8 verlaufen, mit anderen Worten gesagt, entlang einer geometrischen Schnittlinie 16, welche durch die Schnittlinie zwischen der oberen Oberfläche 5 und der unteren Oberfläche 6 in der besagten ersten Ebene E1 gebildet wird.

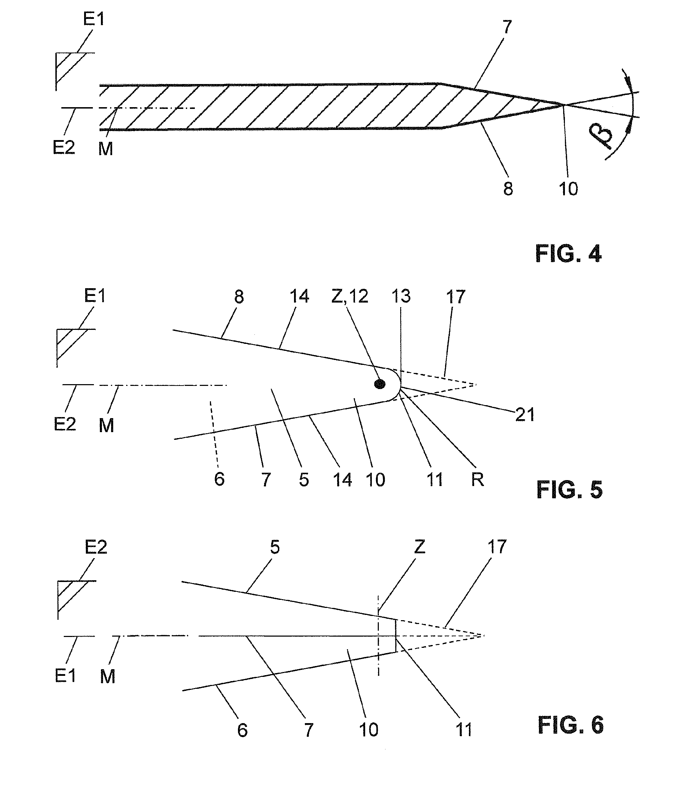

Die erste Schneidkante 7 und die zweite Schneidkante 8 treffen im Bereich des distalen Endes 9 des Schneidenabschnittes 3 aufeinander und bilden eine Spitze 10. Die Spitze 10 ist mit einer Rundung 11, die eine Rundungsfläche 13 bereitstellt, gerundet ausgebildet. Es kann mit anderen Worten von einer verrundeten Spitze 10 gesprochen werden. Diese Rundung 11 kann besonders gut in der schematischen Figur 5 erkannt werden.

Diese verrundete Spitze 10 vereinfacht das Positionieren des Inzisionsmessers 1 auf der Oberfläche des Auges. Dabei kann das Messer über die Bindehaut mit Kontakt zur Bindehaut gezogen werden, ohne einzuhängen, was bei aus dem Stand der Technik bekannten Inzisionsmessern nicht möglich ist. Der Operateur kann dabei, während das Inzisionsmesser 1 über die Bindehaut gezogen wird, den Blutgefässen erfolgreich ausweichen und eine geeignete Stelle finden, um die Inzision anzusetzen. Somit kann eine unnötige Verletzung der Blutgefässe vermieden werden.

Weiter ist eine verrundete Spitze 10 mechanisch stabiler als eine Spitze ohne die Verrundung. Bei einer Spitze, welche ohne die Verrundung ausgebildet ist, laufen die beiden Oberflächen 5, 6 mit den Schneidkanten 7 und 8 direkt aufeinander zu, und es wird im vorderen Bereich des Messers eine im Querschnitt gesehen eine äusserst dünne und geometrische nicht genau bestimmbare spitze Struktur bereitgestellt. Beim Berühren anderer Instrumente während der Handhabung vor dem Eindringen des Messers in die Sklera kann die Spitze dabei verbogen werden, was ein Eindringen erschwert oder gar verunmöglicht. Ein Verbiegen kann durch verrundete Spitze 10 vermieden werden.

Die verrundete Spitze 10 bzw. die Rundung 11 wird vorzugsweise mit einem Schleifprozess hergestellt.

Von der Fig. 2 kann gut erkannt werden, dass sich die Rundung 11 um ein Zentrum 12 herum erstreckt, wobei das Zentrum 12 auf der Mittelachse M in der ersten Ebene E1 liegt. Das Zentrum 12 liegt bezüglich des Querschnittes gesehen also mittig im Instrument 1. Die Rundung 11 weist eine Rundungsfläche 13 auf, die sich im Wesentlichen rechtwinklig zur ersten Ebene E1 erstreckt, so wie dies dann weiter unten noch ausgeführt wird.

Zwecks allgemeiner Definition kann von der Figur 2 und 3 auch erkannt werden, dass zur ersten Ebene E1 eine zweite Ebene E2 vorhanden ist, wobei die beiden Ebenen E1, E2 rechtwinklig zueinander stehen. Die Mittelachse M stellt dabei die Schnittachse der beiden Ebenen E1, E2 dar, das heisst, die beiden Ebenen E1 und E2 verlaufen durch die Mittelachse M.

Anhand der Fig. 2 und der Fig. 5 werden nun weitere Eigenschaften der Rundung 11 weiter erläutert. Die Rundung 11 weist vorzugsweise einen konstanten Radius R auf. Alternativ kann die Rundung 11 auch einen variablen Radius R aufweisen, das heisst, dass die Rundung 11 abschnittsweise mit unterschiedlichen Radien ausgebildet werden kann. Die Rundung 11 ist zudem, wie in der Fig. 5 gut erkannt werden kann, konvex ausgebildet.

Die Rundung 11 bzw. die durch die Rundung 11 bereitgestellte Rundungsfläche 13 erstreckt sich im Wesentlichen rechtwinklig zur ersten Ebene E1. Dies kann in den Fig. 2, 5 und 6 gut erkannt werden. Bezüglich der ersten Ebene E1 erstreckt sich die Rundung 11 beidseitig von besagter Ebene E1, wie dies in der Figur 6 erkennbar ist. Folglich wird der Schleifprozess am Inzisionsmesser 1 im Wesentlichen in der ersten Ebene E1 durchgeführt. Auch denkbar ist eine mehrachsige Bewegung.

Die obere Oberfläche 5 und die untere Oberfläche 6 verlaufen bezüglich der ersten Ebene E1 zur Spitze 10 aufeinander zu. Das heisst, der Querschnitt verkleinert sich vom distalen

Ende 4 gesehen bis hin zur Spitze 10. Die Rundung 11 erstreckt sich dabei, wie bereits erwähnt, im Wesentlichen senkrecht zur ersten Ebene E1.

In den Fig. 5 und 6 ist zudem mit gestrichelten Linien 17 die Form eines Inzisionsmessers 1 ohne die Herstellung der Rundung 11 gezeigt. Hier kann gut erkannt werden, dass ohne den Schleifprozess, durch welchen die Rundung 11 geschaffen wird, die Spitze 10 in eine eigentliche Spitze verläuft. Das heisst, der Querschnitt des Inzisionsmessers 1 im Bereich der Spitze strebt dabei gegen 0 im allervordersten Bereich der Spitze 10. Durch den Schleifprozess werden Teile der Spitze 10 entfernt, wodurch die Rundung 11 geschaffen wird. Die Rundung 11 stellt dabei eine Rundungsfläche 13 bereit. Die Rundungsfläche 13 verläuft hier um eine durch besagtes Zentrum 12 hindurchlaufende und zur ersten Ebene E1 rechtwinklig stehende Zentrumsachse Z. Diese Zentrumsachse Z ist in der Fig. 6 eingezeichnet. In der Fig. 5 verläuft diese Zentrumsachse Z im Wesentlichen senkrecht zur Zeichnungsblattoberfläche bzw. zur eingezeichneten Ebene E1. In diesen beiden Fig. 5 und 6 kann somit gut erkannt werden, dass die Rundungsfläche 13 der Rundung 11 im Wesentlichen rechtwinklig zur ersten Ebene E1 verläuft.

Bezüglich der Rundung 11 kann auch gesagt werden, dass die Rundung 11 beim Herstellprozess durch ein Abtrennen von Teilen der Spitze 10 bereitgestellt wird.

Im Hinblick auf die geometrische Ausbildung kann auch gesagt werden, dass die Rundungsfläche 13 im Bereich der Spitze 10 durch einen virtuellen Zylinder 21 mit kreisrundem oder elliptischem Querschnitt begrenzt wird, wobei die Mittelachse des Zylinders 21 rechtwinklig zur ersten Ebene und zur Mittelachse steht. Vorzugsweise erstreckt sich die Rundungsfläche 13 um eine durch besagtes Zentrum hindurchlaufende und zur ersten Ebene E1 rechtwinklig stehende Zentrumsachse. Das Zentrum wäre dann Teil der Mittelachse des besagten virtuellen Zylinders und die Mittelachse wäre kollinear zur Zentrumsachse.

In einer alternativen, in den vorliegenden Figuren nicht gezeigten, Ausführungsform, kann die Rundung 11 vom besagten Zentrum 12 sich auch kugelförmig oder ellipsoidförmig mit unterschiedlichen Radien erstrecken, so dass die beiden Schneidkanten und teilweise die beiden Oberflächen 5, 6 durch die Rundung 11 abgerundet sind. In Bezug auf die Fig. 6 würde das dann heissen, dass die Rundungsfläche 13 dann nicht mehr in der Seitenansicht als Gerade wahrgenommen wird, sondern dort ebenfalls gerundet wahrnehmbar ist.

Bevorzugterweise liegt der Radius R der Rundung 12 im Bereich von 0.01 bis 0.1 mm. Besonders bevorzugt liegt der Radius R der Rundung im Bereich von 0.01 bis 0.04 mm. Der Durchmesser des Schaftabschnittes 2 liegt vorzugsweise im Bereich von 0.35 bis 0.7 mm.

Von der Fig. 3 kann gut erkannt werden, dass der Scheitel der oberen Oberfläche 5 und der Scheitel der unteren Oberfläche 6 im Querschnitt durch die Mittelachse M gesehen einen Winkel α einschliessen. Der Scheitel der jeweiligen Oberfläche ist dabei im Wesentlichen eine Gerade, welche bei Betrachtung der Oberfläche auf die zweite Ebene erkennbar wird. In der Fig. 3 also auf die Blattebene. Die beiden Oberflächen 5 und 6 bzw. die Scheitel der Oberflächen 5, 6 verlaufen dabei geneigt zur Mittelachse. Der Winkel α ist im Bereich von 1° ° bis 20°, vorzugsweise im Bereich von 8° bis 14°. Der Winkel der jeweiligen Oberfläche 5, 6 zur Mittelachse M ist vorzugsweise gleich. Folglich verläuft die Mittelachse M im Querschnitt gesehen als Winkelhalbierende zwischen den Scheiteln der beiden Oberflächen 5 und 6. Ebenfalls verläuft die Winkelhalbierende in der Ebene E1.

In den Fig. 2 und 3 kann dann weiter erkannt werden, dass die Oberflächen 5 und 6 sich derart aufweiten, dass diese in den Schaftabschnitt 2 übergehen. Mit anderen Worten könnte auch gesagt werden, dass zwischen den Schneidkanten 7, 8 bzw. dem hinteren Ende der Schneidkanten 7, 8 ein Übergangsbereich 18 vorhanden ist, welcher den Schneidenabschnitt 3 an den Schaftabschnitt 2 entsprechend anschliesst. In diesem Übergangsabschnitt 18 weitet sich dann der Querschnitt des Instrumentes zwischen den Oberflächen 5 und 6 auf den Durchmesser des Schaftabschnittes 2 bzw. auf die Mantelfläche 15 auf. Das hintere Ende der Schneidkanten 7, 8, welches bezüglich der Mittelachse gegenüber der Spitze 10 liegt, trägt das Bezugszeichen 19.

Die obere Oberfläche 5 und die untere Oberfläche 6 sind vorzugsweise konvex gerundet ausgebildet, so wie dies im Schnitt A-A in der Fig. 2 erkennbar ist. Der Radius dieser konvexen Oberfläche nimmt in einer Schnittebene A-A, die senkrecht zur besagten Ebene E1 des Schaftabschnittes 2 steht, mit zunehmender Entfernung von der Spitze 10 zu. Die Konvexität wird also mit zunehmender Entfernung von der Spitze 10 entsprechend vergrössert.

Die konvexen Oberflächen 5 und 6 sind dabei derart ausgebildet, dass sich diese von der ersten Schneidkante 7 bis zur zweiten Schneidkante 8 konvex erstrecken. Vorzugsweise ist das geometrische Zentrum der Konvexität ausserhalb des Inzisionsmessers. Mit anderen Worten kann auch gesagt werden, dass die Konvexität einen linsenförmigen Querschnitt bereitstellt. Der eigentliche Radius der konvexen Oberfläche 5, 6 kann abschnittsweise verändert werden, so dass in einer bestimmten Schnittebene die Konvexität unterschiedliche Radien aufweist. Dies kann in der Schnittdarstellung A-A in der Fig. 2 gut erkannt werden. Der Radius im Bereich der beiden Schneidenkanten 7, 8 kann dabei grösser gewählt sein, als beim Scheitelpunkt 20 der konvexen Oberflächen 5, 6.

Von den Fig. 2, 3 und 4 kann dann weiter erkannt werden, dass sich die Schneidkanten 7, 8 jeweils entlang von geometrischen Geraden 14 erstrecken. Von der Spitze 10 her gesehen erstrecken sich dabei diese Schneidkanten 7, 8 winklig zur Mittelachse M. Die Schneidkanten 7, 8 schliessen dabei einen Winkel β ein. Mit anderen Worten kann auch gesagt werden, dass die Schneidkanten 7, 8 mit einem Winkel β winklig zueinander verlaufen. Die Mittelachse M ist dabei vorzugsweise die Winkelhalbierende des Winkels β. Bezüglich der Draufsicht in der Fig. 5 kann auch gesagt werden, dass sich die Geraden 14, welche die Schneidkanten 7, 8 repräsentieren, tangential an die Rundung 11 bzw. an die Rundungsfläche 13 anschliessen. Die Geraden 14 liegen dabei in der ersten Ebene E1.

Der Winkel β zwischen den Schneidkanten 7, 8 liegt vorzugsweise zwischen 10° und 30°. Besonders bevorzugt liegt der Winkel zwischen 15° und 25°, besonders bevorzugt zwischen 17° und 23°.

### BEZUGSZEICHENLISTE

- 1: Inzisionsmesser
- 2: Schaftabschnitt
- 3: Schneidenabschnitt
- 4: distales Ende
- 5: obere Oberfläche
- 6: untere Oberfläche
- 7: erste Schneidkante
- 8: zweite Schneidkante
- 9: distales Ende
- 10: Spitze
- 11: Rundung
- 12: Zentrum
- 13: Rundungsfläche
- 14: Gerade
- 15: Mantelfläche
- 16: Schnittlinie
- 17: gestrichelte Linie
- 18: Übergangsbereich
- 19: hinteres Ende
- 20: Scheitelpunkt
- 21: Zylinder
- E1: erste Ebene
- E2: zweite Ebene
- M: Mittelachse
- R: Radius
- Z: Zentrumsachse

## Patentansprüche

1. Inzisionsmesser (1) für die Ophthalmologie umfassend
einen Schaftabschnitt (2) mit einem im Wesentlichen zylindrischen Querschnitt, welcher sich entlang einer Mittelachse (M) erstreckt, und
einen Schneidenabschnitt (3), der sich am distalen Ende (4) des Schaftabschnittes (2) an den Schaftabschnitt (2) anschliesst, wobei der Schneidenabschnitt (3) eine obere Oberfläche (5) und eine untere Oberfläche (6) umfasst, wobei die obere Oberfläche (5) und die untere Oberfläche (6) eine erste Schneidkante (7) und eine zweite Schneidkante (8) in einer ersten Ebene (E1) bilden, wobei die erste Schneidkante (7) und die zweite Schneidkante (8) im Bereich des distalen Endes (9) des Schneidenabschnittes (3) aufeinander treffen und eine Spitze (10) bilden, **dadurch gekennzeichnet,**
**dass** die Spitze (10) mit einer Rundung (11) abgerundet ausgebildet ist.

2. Inzisionsmesser (1) nach Anspruch 1, **dadurch gekennzeichnet, dass** sich die Rundung (11) um ein Zentrum (12) herum erstreckt, wobei das Zentrum (12) auf der Mittelachse (M) und in der ersten Ebene (E1) liegt.

3. Inzisionsmesser (1) nach einem der vorhergehenden Ansprüche, **dadurch gekennzeichnet, dass** die Rundung (11) einen konstanten Radius (R) oder einen variablen Radius aufweist und/oder dass die Rundung (11) konvex ausgebildet ist.

4. Inzisionsmesser (1) nach einem der vorhergehenden Ansprüche, **dadurch gekennzeichnet, dass** sich die Rundung (11) eine Rundungsfläche (13) aufweist, die sich im Wesentlichen rechtwinklig zur ersten Ebene (E1) erstreckt, wobei sich die Rundung beidseitig zur ersten Ebene (E1) erstreckt.

5. Inzisionsmesser (1) nach Anspruch 4, **dadurch gekennzeichnet, dass** die Rundungsfläche (13) sich um eine durch besagtes Zentrum (12) hindurchlaufende und zur ersten Ebene (E1) rechtwinklig stehende Zentrumsachse (Z) erstreckt.

6. Inzisionsmesser (1) nach einem der vorhergehenden Ansprüche, **dadurch gekennzeichnet, dass** die obere Oberfläche (6) und die untere Oberfläche (7) bezüglich der ersten Ebene (E1) zur Spitze (10) aufeinander zulaufen, wobei sich die Rundung (11) im Wesentlichen senkrecht zur ersten Ebene (E1) erstreckt.

7. Inzisionsmesser (1) nach einem der Ansprüche 1 bis 3, **dadurch gekennzeichnet, dass** sich Rundung (11) von besagtem Zentrum (12) kugelförmig oder ellipsoidförmig mit unterschiedlichen Radien erstreckt, so dass die beiden Schneidkanten (7, 8) und teilweise die beiden Oberfläche (5, 6) durch die Rundung (11) abgerundet sind.

8. Inzisionsmesser (1) nach einem der vorhergehenden Ansprüche, **dadurch gekennzeichnet, dass** der Radius (R) der Rundung (11) im Bereich von 0.01 bis 0.10 mm, besonders bevorzugt im Bereich von 0.01 bis 0.04 mm liegt und dass der Durchmesser des Schaftabschnittes (2) im Bereich von 0.35 bis 0.7 mm liegt.

9. Inzisionsmesser (1) nach einem der vorhergehenden Ansprüche, **dadurch gekennzeichnet, dass** der Scheitel der oberen Oberfläche (5) und der Scheitel der unteren Oberfläche (6) auf eine zur ersten Ebene (E1) rechtwinklig stehende zweite Ebene (E2) gesehen einen Winkel (α) einschliessen und geneigt zueinander sowie zur Mittelachse (M) verlaufen, wobei der Winkel (α) im Bereich von 1° bis 20°, vorzugsweise im Bereich von 8° bis 14° verläuft und wobei der Winkel der jeweiligen Oberflächen (5, 6) zur Mittelachse (M) vorzugsweise gleich ist.

10. Inzisionsmesser (1) nach einem der vorhergehenden Ansprüche, **dadurch gekennzeichnet, dass** die obere Oberfläche (5) und die untere Oberfläche (6) in den Schaftabschnitt (2) übergehen.

11. Inzisionsmesser (1) nach einem der vorhergehenden Ansprüche, **dadurch gekennzeichnet, dass** die obere Oberfläche (5) und die untere Oberfläche (6) im Querschnitt gesehen konvex gerundet ausgebildet sind, wobei der Radius in einer Schnittebene (A-A), die senkrecht zur besagten ersten Ebene (E1) und zur Mittelachse (M) des Schaftabschnittes (2) steht, mit zunehmender Entfernung von der Spitze (10) vorzugsweise grösser wird.

12. Inzisionsmesser (1) nach Anspruch 11, **dadurch kennzeichnet, dass** sich die Oberflächen (5, 6) sich von der ersten Schneidkante (7) bis hin zur zweiten Schneidkante (8) konvex erstrecken und/oder dass das geometrische Zentrum der Konvexität ausserhalb des Inzisionsmessers (1) liegt und/oder dass der Radius der konvexen Oberfläche abschnittsweise verändert ist.

13. Inzisionsmesser (1) nach einem der vorhergehenden Ansprüche, **dadurch gekennzeichnet, dass** die Schneidkanten (7, 8) sich entlang jeweils einer Geraden (14) erstrecken, wobei die Gerade (14) winklig zur Mittelachse (M) steht, so dass die Schneidkanten (7, 8) mit einem Winkel (β) winklig zueinander verlaufen.

14. Inzisionsmesser (1) nach Anspruch 13, **dadurch gekennzeichnet, dass** sich die Geraden (14) und somit die Schneidkanten (7, 8) in einer Ansicht rechtwinklig auf die Ebene (E1) gesehen sich tangential an die Rundung (11) bzw. die Rundungsfläche (13) anschliessen.

15. Inzisionsmesser (1) nach Anspruch 13, **dadurch gekennzeichnet, dass** der Winkel (β) zwischen den Schneidkanten (7, 8) zwischen 10° und 30° liegt, insbesondere zwischen 15° und 25° besonders bevorzugt zwischen 17° und 23° liegt und wobei die Mittelachse (M) mittig zwischen den Schneidkanten (7, 8) verläuft.
